# EUROPEAN PATENT SPECIFICATION

(11) **EP 3 210 649 B1**
(45) Date of publication and mention of the grant of the patent: **04.09.2019**
(21) Application number: 14904353.1
(22) Date of filing: 07.11.2014
(51) Int. Cl.: A61N 7/00, A61N 7/02

(54) **ULTRASONIC CARTRIDGE AND ULTRASONIC TREATMENT HEAD USING SAME**
ULTRASCHALLSONDE UND ULTRASCHALLKOPF DAMIT
CARTOUCHE À ULTRASONS ET TÊTE DE TRAITEMENT PAR ULTRASONS L'UTILISANT

(30) Priority: 24.10.2014 KR 20140145219
(43) Date of publication of application: 30.08.2017
(73) Proprietor: CLASSYS INC., Gangnam-gu Seoul 135-878 (KR)
(72) Inventor: JUNG, Sung Jae, Seoul 137-810 (KR)
(74) Representative: Cabinet Laurent & Charras
(86) International application number: PCT/KR2014/010701
(87) International publication number: WO 2016/064016

(56) References cited:
- JP-A- 2009 204 328
- KR-A- 20060 121 267
- KR-A- 20110 009 337
- KR-A- 20120 040 909
- KR-B1- 101 177 691
- KR-B1- 101 177 691
- KR-B1- 101 307 551
- US-A1- 2011 072 970
- US-A1- 2014 155 747

## Description

### Technical Field

The present invention relates to a cartridge and a head that are used for obesity treatment using HIFU (High Intensity Focused Ultrasound); and, more particularly, to an ultrasonic cartridge that can improve obesity treatment effects because it can perform uniform scanning even using 2-axis control, and an ultrasonic treatment head using the ultrasonic cartridge.

### Background Art

Recently, obesity has rapidly increased with westernization of dietary habits and it has become a major health and beauty concern for many people. Consequently, various diet programs and ultrasonic obesity treatment devices have been developed and are generally used to treat obesity.

HIFU (High Intensity Focused Ultrasound) has been used for anticancer treatment, whereby cancer cells are destroyed by selectively solidifying tumors in internal organs at high temperature in a non-invasive way, but Solta Medical Inc. in U.S. has developed Liposonix, which is based on HIFU, to treat abdominal obesity. The method of removing fat uses the phenomenon that when ultrasonic waves are focused on a predetermined position of a fat cell, the temperature of a tissue rapidly increases up to 65 ∼ 100°C and the tissue is destroyed. HIFU equipment, unlike other dermatologic equipment, for example, laser and RF high-frequency equipment, induces coagulation necrosis of fat by concentrating HIFU energy on a selected portion in a non-invasive way without any damage to the skin. The necrotized fat cell is naturally removed by restoring mechanism for injured parts of our bodies.

As an ultrasonic obesity treatment apparatus, there is an "Apparatus and methods for the destruction of adipose tissue" disclosed in Korean Patent Application No. 10-2007-7027898. The apparatus disclosed in Korean Patent Application Publication No. 10-2007-7027898 is composed of at least a scan head having one ultrasonic transducer, a transducer in order to treat obesity in a non-invasive way by deforming the tissue of subcutaneous fat with ultrasonic energy. Further, a "Tissue imaging and treatment system", which has been disclosed in Korean Patent Application No. 10-2011-7017983, includes a hand wand that is manually operated and a detachable emitter-receiver module having an ultrasonic transducer. The system displays skin images in an imaging mode through a display connected to the hand wand and makes skin elastic and removes wrinkles by radiating ultrasonic waves to the portion of 3∼4mm (SMAS layer) under the skin in a treating mode.

Meanwhile, those ultrasonic obesity treatment apparatuses include a head or a handpiece that is controlled by a controller to scan subcutaneous fat of a patient by generating HIFU. A "Ultrasound therapy head with movement control" disclosed in Korean Patent Application No. 10-2006-7013272 includes an enclosure having a window, an HIFU transducer suspended in the enclosure, and an actuator moving the HIFU transducer in X-axial direction and Y-axial direction so that the HIFU transducer scans subcutaneous fat through the window using HIFU. Further, a "Therapy head for use with an ultrasound system" disclosed in Korean Patent Application No. 10-2010-7019552 includes an enclosure, a partition separating a lower compartment with an window and an upper compartment, an opening of the partition, a control arm disposed in the opening and being movable, an operation assembly disposed in the upper compartment and controlling the control arm, and an HIFU transducer coupled to the lower end of the control arm.

Further, a "3 axis control head of therapy system for treatment of fatness using HIFU" disclosed in Korean Patent No. 10-1177691 by the applicant(s) is configured to be controlled by a controller to three-dimensionally scan fat layers of a patient by controlling three axes.

Document KR10-2006-0121267 A discloses a therapy head for use in HIFU procedures provided with a controller to identify locations where the therapy head should radiate energy into a patient.

Document KR 2012 0040909 discloses a 3D-transfer type hand piece for a high intensive focused ultrasonic device and document KR 101 307 551 B1 discloses a device for an ultrasonic device including handpiece and a detachable cartridge.

### Disclosure

### Technical Problem

There is a need for a process of scanning fat layers of a patient using a head in obesity treatment using high intensity focused ultrasound, but there is a problem in the related art that scanning is performed along a curve (an arc) in scanning by a head due to the characteristics of pivoting, so energy decreases and a focus depth changes, as it goes to the outer side, so scanning is not uniform.

The present invention has been made in an effort to solve the problem and an object of the present invention is to provide an ultrasonic cartridge that can improve treatment effects through uniform scanning and an ultrasonic treatment head using the ultrasonic cartridge.

### Technical Solution

The scope of the invention is defined in appended independent claim 1, preferred embodiments are described in the dependent claims.

In order to achieve the objects of the present invention, an ultrasonic treatment head includes: a head body configured to be easily held by a user's hand and put on the skin of a patient for treatment; and a cartridge detachably coupled to the head body and performing in-plane scanning on a treatment area by generating ultrasonic waves in accordance with operation of the head body.

The head body may include: a body housing for supporting and protecting internal components and providing an interface for a user; a first actuator generating reciprocation on a first axis; a second actuator generating reciprocation on a second axis, and a stage pivoted about a fixed axis by the first actuator and the second actuator. The head body may further include a controller for controlling the first actuator and the second actuator.

The first actuator and a second actuator each may include: a motor; a lead screw; a coupler for coupling a rotary shaft of the motor to the lead screw; a support frame for supporting the lead screw; a slider reciprocating with rotation of the lead screw; a position detector for detecting a position of the slider; and a ball joint disposed at a free end of the slider.

Further, in order to achieve the objects of the present invention, an ultrasonic cartridge includes: a cartridge housing for keeping a transducer and liquid therein; a flat window disposed on the cartridge housing to transmit ultrasonic waves to a treatment area; a body coupler for coupling to the head body; an ultrasonic transducer generating ultrasonic waves in response to an operation signal input through the body coupler; and a fitting assembly moving the ultrasonic transducer on the flat window for in-plane scanning by a pivot motion, which is transmitted through the body coupler, of the stage.

The fitting assembly includes: a first support having a hole therein and coupled to the body coupler; a compression spring inserted in the hole of the first support; a second support inserted in the hole of the first support to compress the compression spring; and a ball joint coupled to the second support. The fitting assembly further includes a transducer housing combined with the ball joint.

The body coupler may include: a controller PCB connected to a controller through a lead connector; a bellows allowing for movement and preventing leakage of the liquid; a cartridge coupler coupled to a stage; and a coupling magnet.

### Advantageous Effects

According to the present invention, a pivot motion of the stage by the first actuator and the second actuator is converted into in-plane motion by the fitting assembly, thereby in-plane scanning is possible. Therefore, uniform energy can be applied to a treatment portion at a desired depth and treatment effects can be improved.

### Description of Drawings

FIG. 1 is a perspective view showing a head of an ultrasonic treatment apparatus according to the present invention.
FIG. 2 is a side view showing a side of the head shown in FIG. 1.
FIG. 3 is a side view showing another side of the head shown in FIG. 1.
FIG. 4 is an exploded perspective view of a head body having an operation assembly in the head according to the present invention.
FIG. 5 is an exploded perspective view of a cartridge that is mounted on the head body according to the present invention.

### Mode for Invention

The present invention and the objects that are achieved by embodiments of the present invention will be made clear from preferred embodiments of the present invention to be described hereafter. The following embodiment are just examples for describing the present invention and do not limit the scope of the present invention.

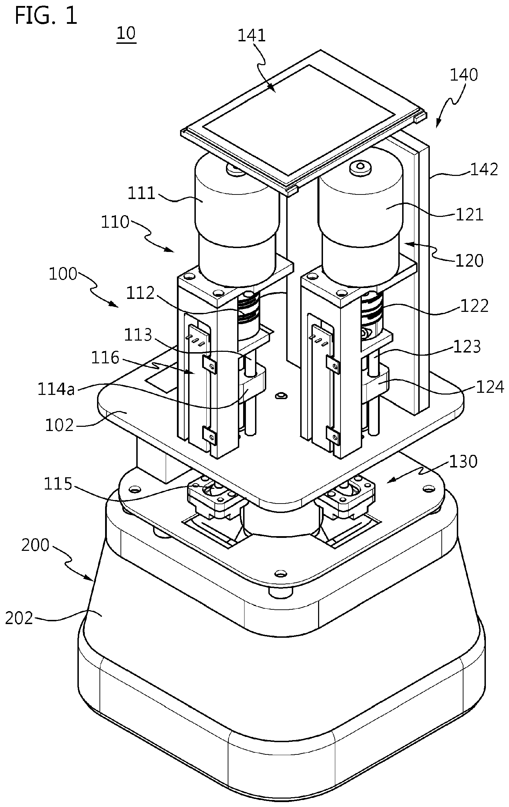

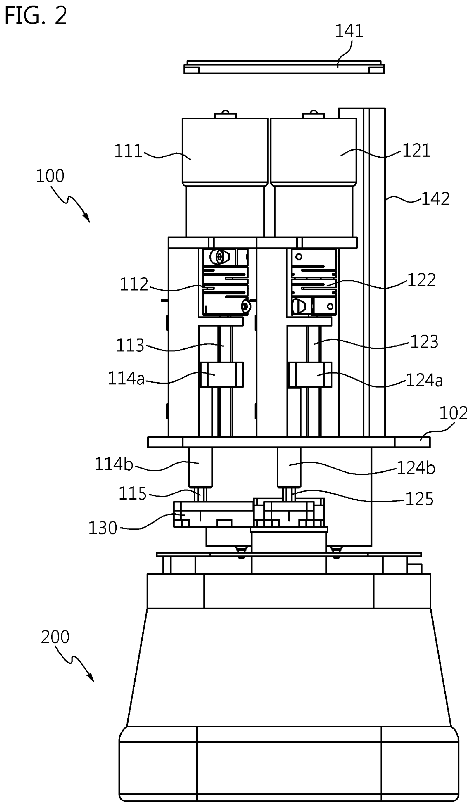

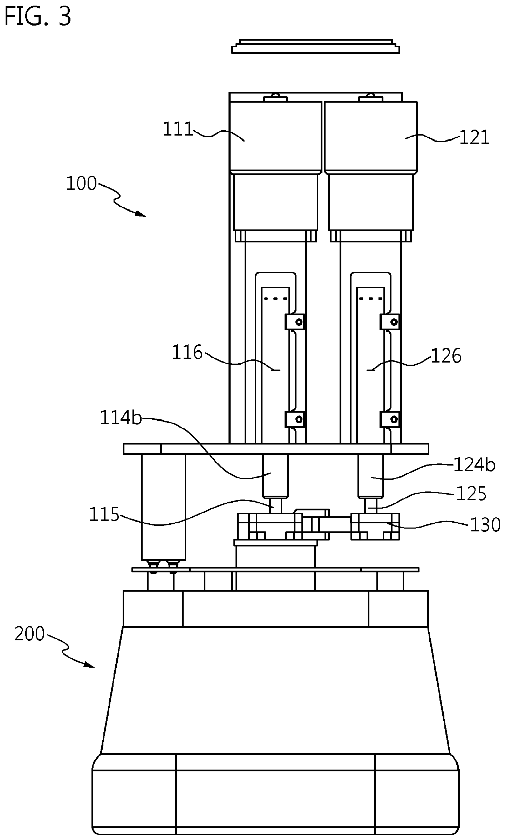

FIG. 1 is a perspective view showing a head of an ultrasonic treatment apparatus according to the present invention, FIG. 2 is a side view showing a side of the head shown in FIG. 1, and FIG. 3 is a side view showing another side of the head shown in FIG. 1.

An ultrasonic treatment head 10 according to the present invention includes a head body 100 having a shape that is easily held by a user's hand and put on the skin of a patient for treatment and a cartridge 200 detachably coupled to the head body 100 and performing in-plane scanning on a treatment area by generating ultrasonic waves in accordance with operation of the head body 100.

When the ultrasonic treatment head 10 according to the present invention is a portable independent part, it can be independently operated by controller in the head body when power is supplied, but when a separate controller is provided, the head may be connected with an external controller through a cable or wirelessly.

Referring to FIGS. 1 to 3, the ultrasonic treatment head 10 according to the present invention is composed of the head body 100 and the cartridge 200 detachably coupled to the head body 100. The head body 100 includes a body housing (not shown) for supporting and protecting internal components and providing an interface for a user, a first actuator 110 generating translation (reciprocation) on a first axis, a second actuator 120 generating translation (reciprocation) on a second axis, and a stage 130 pivoted about a fixed axis by the first actuator 110 and the second actuator 120.

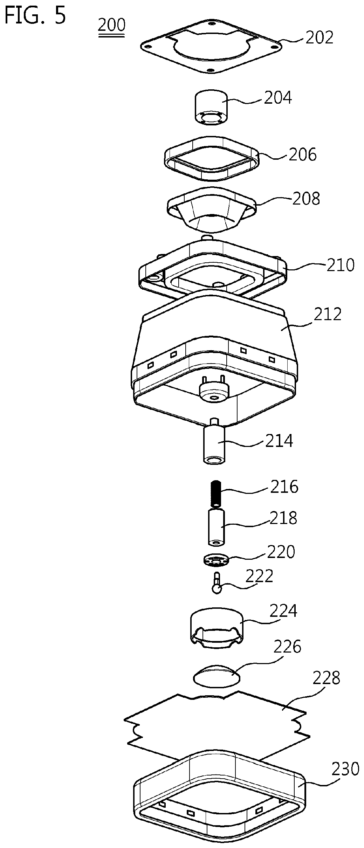

The cartridge 200, which will be described in detail below, includes a cartridge housing 202 for keeping a transducer and liquid therein, a flat window 228 (see FIG. 5) disposed on the cartridge housing to transmit ultrasonic waves to a treatment area, a body coupler for coupling to the head body, an ultrasonic transducer 226 generating ultrasonic waves in response to an operation signal input through the body coupler, and a fitting assembly moving the ultrasonic transducer 226 on the flat window 228 for in-plane scanning by a pivot motion, which is transmitted through the body coupler, of the stage.

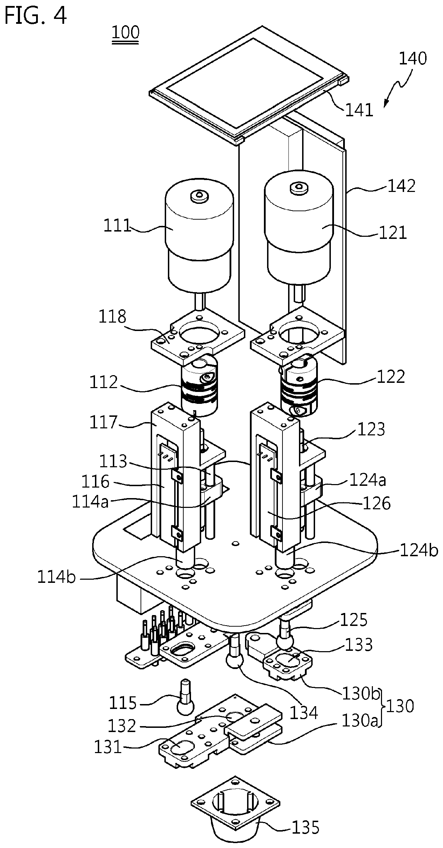

FIG. 4 is an exploded perspective view of a head body having an operation assembly in the head according to the present invention.

Referring to FIG. 4, the first actuator 110 generating translation (reciprocation) on the first axis includes: a BLDC motor (111); a lead screw 113, a coupler 112 for coupling a rotary shaft of the BLDC motor 11 to the lead screw 113; a support frame 117 for supporting the lead screw 113; a slider 114 reciprocating with rotation of the lead screw; a position resistor 116 for detecting the position of the slider 114, and a ball joint 115 disposed at a free end of the slider 114.

Further, the second actuator 120 generating translation (reciprocation) on the second axis, same as the first actuator, includes: a BLDC motor 121; a lead screw 123; a coupler 122 for coupling to a rotary shaft of the BLDC motor to the lead screw 123; a support frame 127 for supporting the lead screw; a slider 124 reciprocating with rotation of the lead screw; a position resistor 126 for detecting the position of the slider; and a ball joint 125 disposed at a free end of the slider.

The stage 130 has three holes 131, 132, and 133 for coupling a fixed ball joint 134 fastened to a base plate 102, a first axis ball joint 115, and a second axis ball joint 125 on the top and has a coupling member 135 for coupling to the cartridge 200 on the bottom. Though not shown in detail in the drawings, the stage 130 is firmly coupled to the cartridge by a magnet so that when the stage 130 is operated, the fitting assembly of the cartridge can move a transducer.

Further, the head body 100 includes a display or a touch screen 141 mounted on the body housing to provide an interface for a user and a control board 142 controlling operation of the first actuator 110 and the second actuator and operating the transducer by executing software programmed in accordance with a predetermined scan algorism. The position resistors 116 and 126 of the actuators are connected to the control board 142, and change the rotational directions of the actuators when the sliders 114 and 124 reach an upper limit or a lower limit.

Other than the components described above, a sensor for checking whether the cartridge 200 has been normally coupled may be additionally mounted on the head body 100.

FIG. 5 is an exploded perspective view of a cartridge that is mounted on the head body according to the present invention.

Referring to FIG. 5, the cartridge 200 includes a cartridge PCB 202, a cartridge coupler 204, a bellows support spring 206, a bellows 208, a top cover 210, a cartridge housing 212, a first support 214, a compression spring 216, a second support 218, a disc 220, a ball joint 222, a transducer housing 224, a transducer 226, a flat window 228, and a bottom cover 230.

The cartridge PCB 202 and the cartridge coupler 204 form a body coupling unit for coupling to the head body 100 and are fastened to a contact connector and a coupling portion of the head body 110 to connect a pivot motion of the stage 130 and electrical signals from the control board 142.

The bellows 208 is coupled to the cartridge housing 212 through the bellows housing 206 to allow movement of the transducer therein and prevents liquid therein from leaking and the flat window 228 is mounted on the cartridge housing 212 to transmit ultrasonic waves to a treatment area. The top cover 210 and the bottom cover 230 are fastened to the cartridge housing 212 and seal the cartridge in cooperation with the bellows 208 and the window 228 to prevent leakage of liquid that is a medium for transmitting ultrasonic waves.

The fitting assembly is composed of the first support 214 having a hole therein and coupled to the body coupler, the compression spring 226 inserted in the hole of the first support 214, a second support 218 inserted in the hole of the first support 214 to compress the compression spring 216, the ball joint 222 coupled to the second support, and the transducer housing 224 combined with the ball joint 222. The fitting assembly moves the ultrasonic transducer 226 for in-plane scanning along the flat window 228 by a pivot motion, which is transmitted through the body coupler, of the stage due to ball joint-coupling of the transducer housing. The ultrasonic transducer 226 is fastened to the transducer housing 224 and generates an ultrasonic wave when an operation signal is input through the body coupler.

The operation of the ultrasonic treatment head according to the present invention having the configuration described above is described hereafter.

In general, the depth of a focus area depends on the treatment purpose in ultrasonic treatment, so a cartridge 200 suitable for the treatment purpose is selected and mounted on the head body 100. Further, it is possible to select an appropriate scan pattern or operation time, depending on the treatment portion or purpose.

When the ultrasonic treatment head 10 is operated through the user interface of the ultrasonic treatment head, the controller 140 starts the first actuator 110 and the second actuator 120 and operates the transducer 226 in accordance with a preprogrammed scan pattern to generate ultrasonic waves or high intensity focused ultrasound.

When the BLDC motor 111 of the first actuator is operated, the lead screw 113 is rotated through the coupler 112 and a slider nut 114a coupled to the lead screw is moved downward on the lead screw 113, so a slider rod 114b integrally connected to the slider nut 114a and the ball joint 115 connected to the slider rod 114b are moved downward. As the ball joint 115 is moved downward, the stage 130 combined with the ball joint pivots on the fixed ball joint 134.

When the second actuator 120 is operated in the same way, the stage 130 pivots in various directions due to combination of a pivot motion by the first axis ball joint and a pivot motion by the second axis ball joint.

When it is detected through the position resistor 116 that the slider 114 of the first actuator has reached a lower limit, the controller 140 changes the rotational direction of the first actuator in the opposite direction, and accordingly, the slider 114 is moved upward. When it is detected through the position resistor 116 that the slider 114 of the first actuator has reached an upper limit in the same way, the controller 140 changes again the rotational direction of the first actuator 110 back into the opposite direction for downward movement, so the slider 114 pushes or pulls the stage 130 by reciprocating between the upper limit and the lower limit.

The pivot motion of the stage 130 is transmitted to the fitting assembly of the cartridge 200 and the fitting assembly moves the transducer 226 at the end on the flat window 228 by the pivot motion. The fitting assembly is changed in the entire axial length by the compression spring 216, so it allows for in-plane scanning by converting the pivot motion into in-plane movement. That is, the fitting assembly is contracted and the spring 216 is compressed much at the center of the flat window 228, and as the fitting assembly moves outward, the compression spring 216 is decompressed and the length of the fitting assembly is stretched, so in-plane scanning is performed in the flat window area. Accordingly, the ultrasonic focus depth of the transducer is made uniform even at the center and the edge in a plane.

## Claims

1. An ultrasonic treatment head comprising:
a head body (100) configured to be easily held by a user's hand and put on skin of a patient for treatment; and
a cartridge (200) detachably coupled to the head body and performing in-plane scanning on a treatment area by generating ultrasonic waves in accordance with operation of the head body;
**characterized in that** the cartridge includes:
a cartridge housing (202) for keeping a transducer (226) and liquid therein;
a flat window (228) disposed on the cartridge housing (202) to transmit ultrasonic waves to a treatment area;
a body coupler for coupling to the head body;
an ultrasonic transducer (226) generating ultrasonic waves in response to an operation signal input through the body coupler; and
a fitting assembly moving the ultrasonic transducer on the flat window for in-plane scanning by a pivot motion, which is transmitted through the body coupler, of the stage, wherein the fitting assembly includes:
a first support (214) having a hole therein and coupled to the body coupler;
a compression spring (216) inserted in the hole of the first support (214);
a second support (218) inserted in the hole of the first support (214) to compress the compression spring (216);
a ball joint (222) coupled to the second support (218) and
a transducer housing (224) combined with the ball joint (222), wherein the ultrasonic transducer (226) is fastened to the transducer housing (224).

2. The ultrasonic treatment head of claim 1, wherein the head body (100) includes:
a body housing for supporting and protecting internal components and providing an interface for a user;
a first actuator (110) generating reciprocation on a first axis;
a second actuator (120) generating reciprocation on a second axis, and
a stage (130) pivoted about a fixed axis by the first actuator (110) and the second actuator (120).

3. The ultrasonic treatment head of claim 2, wherein the head body (100) further includes a controller for controlling the first actuator (110) and the second actuator (120).

4. The ultrasonic treatment head of claim 2 or 3, wherein the first actuator (110) and a second actuator (120) each include:
a motor (111);
a lead screw (113);
a coupler (112) for coupling a rotary shaft of the motor (111) to the lead screw (113);
a support frame (127) for supporting the lead screw (113);
a slider (124) reciprocating with rotation of the lead screw (113);
a position detector (126) for detecting a position of the slider (124); and
a ball joint (125) disposed at a free end of the slider (124).

5. The ultrasonic treatment head of claim 1, wherein the body coupler includes:
a controller PCB (202) connected to a controller through a lead connector;
a bellows (208) allowing for movement and preventing leakage of the liquid;
a cartridge coupler (204) coupled to a stage (130); and
a coupling magnet.

## Patentansprüche

1. Ein Ultraschallbehandlungskopf umfassend
einen Gerätekörper (100), das dazu konfiguriert ist, gut in die Hand des Benutzers zu passen, um das Gerät während der Behandlung über die Haut des Patienten zu führen; und
ein Steckmodul (200), das mit dem Gerätekörper zur Durchführung eines Scans in der Ebene, in einem Behandlungsbereich, durch Erzeugung von Ultraschallwellen entsprechend der Bedienung des Gerätekörpers lösbar verbunden ist;
**dadurch gekennzeichnet, dass** das Steckmodul umfasst:
ein Steckmodulgehäuse (202) zur Unterbringung eines Wandlers (226) und von Flüssigkeit;
ein Sichtfenster (228), das am Steckmodulgehäuse (202) zur Übertragung von Ultraschallwellen auf den Behandlungsbereich angebracht ist;
einen Gerätekoppler zum Verbinden mit dem Gerätekörper;
einen Ultraschallwandler (226), der Ultraschallwellen in Reaktion auf ein durch den Gerätekoppler eingegebenes Betriebssignal erzeugt; und
eine Montagevorrichtung, die den Ultraschallwandler auf dem Sichtfenster bewegt, um durch eine Schwenkbewegung einen Scan in der Ebene durchzuführen, übertragen über den Gerätekoppler, der Grundplatte, wobei die Montagevorrichtung umfasst:
eine erste Halterung (214) mit einem Loch, die mit dem Gerätekoppler verbunden ist;
eine Druckfeder (216), die in das Loch der ersten Halterung (214) eingeführt ist, eine zweite Halterung (218), die in das Loch der ersten Halterung (214) eingeführt ist,
um die Druckfeder (216) zusammenzudrücken;
ein Kugelgelenk (222), das mit der zweiten Halterung (218) verbunden ist und
ein Wandlergehäuse (224), das mit dem Kugelgelenk (222) kombiniert ist, wobei der Ultraschall-Wandler (226) an dem Wandlergehäuse (224) befestigt ist,

2. Ultraschallbehandlungskopf nach Anspruch 1, wobei der Gerätekörper (100) umfasst:
ein Gerätegehäuse zum Halten und Stützen innerer Komponenten und zur Bereitstellung eines Interface für den Benutzer;
ein erstes Stellglied (110), das eine Hin- und Herbewegung auf einer ersten Achse erzeugt;
ein zweites Stellglied (120), das eine Hin- und Herbewegung auf einer zweiten Achse erzeugt, und
eine Grundplatte (130), die durch das erste Stellglied (110) und das zweite Stellglied (120) um eine feste Achse geschwenkt wird.

3. Ultraschallbehandlungskopf nach Anspruch 2, wobei der Gerätekörper (100) weiterhin einen Regler zur Steuerung des ersten Stellgliedes (110) und des zweiten Stellgliedes (120) umfasst.

4. Ultraschallbehandlungskopf nach Anspruch 2 oder 3, wobei das erste Stellglied (110) und das zweite Stellglied (120) jeweils umfassen:
einen Motor (111);
eine Leitspindel (113);
einen Koppler (112), um eine Drehachse des Motors (111) mit der Leitspindel (113) zu verbinden,
einen Halterahmen (127) zum Halten der Leitspindel (113);
einen Schieber (124), der sich mit der Drehung der Leitspindel (113) hin- und herbewegt;
einen Positionsmelder (126) zum Erkennen einer Position des Schiebers (124) und
ein Kugelgelenk (125), das am freien Ende des Schiebers angeordnet ist.

5. Ultraschallbehandlungskopf nach Anspruch 1, wobei der Gerätekoppler umfasst:
einen Regler PCB (202), der über eine Steckverbindung mit einem Regler verbunden ist;
einen Balg (208), der die Bewegung ermöglicht und ein Auslaufen der Flüssigkeit verhindert;
einen Steckmodulkoppler (204), der mit einer Grundplatte (130) verbunden ist und
einen Verbindungsmagnet.

## Revendications

1. Tête de traitement par ultrasons comprenant :
un corps de tête (100) configuré pour être facilement tenu par la main d'un utilisateur et placé sur la peau d'un patient pour un traitement ; et
une cartouche (200) couplée de manière amovible au corps de la tête et effectuant un balayage dans le plan sur une zone de traitement en générant des ondes ultrasonores en fonction du fonctionnement du corps de la tête ;
**caractérisée en ce que** la cartouche comprend :
un boîtier de cartouche (202) destiné à contenir un transducteur (226) et un liquide en son sein ;
une fenêtre plate (228) disposée sur le boîtier de cartouche (202) pour transmettre des ondes ultrasonores à une zone de traitement ;
un coupleur de corps destiné à être couplé au corps de tête ;
un transducteur ultrasonore (226) générant des ondes ultrasonores en réponse à un signal de fonctionnement entré par l'intermédiaire du coupleur de corps ; et
un ensemble de raccord déplaçant le transducteur ultrasonore sur la fenêtre plate pour un balayage dans le plan par un mouvement de pivotement, qui est transmis à travers le coupleur de corps, de la platine, l'ensemble de raccord comprenant :
un premier support (214) comportant un trou en son sein et couplé au coupleur de corps ;
un ressort de compression (216) inséré dans le trou du premier support (214) ;
un second support (218) inséré dans le trou du premier support (214) pour comprimer le ressort de compression (216) ;
un joint à rotule (222) couplé au second support (218) et un boîtier de transducteur (224) associé au joint à rotule (222), dans laquelle le transducteur ultrasonore (226) est fixé au boîtier de transducteur (224).

2. Tête de traitement par ultrasons selon la revendication 1, dans laquelle le corps de tête (100) comprend :
un boîtier de corps pour supporter et protéger des composants internes et fournir une interface pour un utilisateur ;
un premier actionneur (110) générant un mouvement de va-et-vient sur un premier axe ;
un second actionneur (120) générant un mouvement de va-et-vient sur un second axe, et
un étage (130) pivoté autour d'un axe fixe par le premier actionneur (110) et le second actionneur (120).

3. Tête de traitement par ultrasons selon la revendication 2, dans laquelle le corps de tête (100) comprend en outre un dispositif de commande pour commander le premier actionneur (110) et le second actionneur (120).

4. Tête de traitement par ultrasons selon la revendication 2 ou 3, dans laquelle le premier actionneur (110) et un second actionneur (120) comprennent chacun :
un moteur (111) ;
une vis-mère (113) ;
un coupleur (112) pour coupler un arbre rotatif du moteur (111) à la vis mère (113) ;
un cadre de support (127) pour supporter la vis-mère (113) ;
un coulisseau (124) se déplaçant en va-et-vient avec une rotation de la vis mère (113) ;
un détecteur de position (126) pour détecter une position du coulisseau (124) ; et
un joint à rotule (125) disposé à une extrémité libre du coulisseau (124).

5. Tête de traitement par ultrasons selon la revendication 1, dans laquelle le coupleur de corps comprend :
une carte de circuit imprimé, PCB, de dispositif de commande (202) connectée à un dispositif de commande via un connecteur de branchement ;
un soufflet (208) permettant un déplacement et empêchant une fuite du liquide ;
un coupleur de cartouche (204) couplé à une platine (130) ; et
un aimant de couplage.
